(19) Europäisches Patentamt / European Patent Office / Office européen des brevets

(11) **EP 4 412 519 B1**

(12) **EUROPEAN PATENT SPECIFICATION**

(45) Date of publication and mention
of the grant of the patent:
**21.01.2026  Bulletin 2026/04**

(21) Application number: **22782548.6**

(22) Date of filing: **28.09.2022**

(51) International Patent Classification (IPC):
*A61B 5/107* (2006.01)      *G01B 5/20* (2006.01)
*G01B 5/207* (2006.01)      *G01B 11/24* (2006.01)
*G06T 7/50* (2017.01)

(52) Cooperative Patent Classification (CPC):
**A61B 5/1072; A61B 5/1073; A61B 5/1075;
A61B 5/1079; G01B 5/207; G06T 7/571;**
A61B 2562/0233; A61B 2562/046; G01B 11/24

(86) International application number:
**PCT/EP2022/077012**

(87) International publication number:
**WO 2023/057283 (13.04.2023 Gazette 2023/15)**

(54) **SURFACE PROFILER**

OBERFLÄCHENPROFILER

PROFILEUR DE SURFACE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.10.2021  GB 202114406**

(43) Date of publication of application:
**14.08.2024  Bulletin 2024/33**

(73) Proprietor: **Verinnogen Ltd
Cambridge Cambridgeshire CB21 4YT (GB)**

(72) Inventors:
• **JOHNSON, Timothy Isaac
Cambridge Cambridgeshire CB21 4YT (GB)**

• **GREEN, Alan
Cambridge Cambridgeshire CB21 4YT (GB)**
• **IRVINE, Michael
Cambridge Cambridgeshire CB21 4YT (GB)**
• **HAZELL, Andrew
Cambridge Cambridgeshire CB21 4YT (GB)**

(74) Representative: **Marks & Clerk LLP
15 Fetter Lane
London EC4A 1BW (GB)**

(56) References cited:
**US-A- 6 125 338    US-A- 6 160 264**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field

The present invention generally relates to an imaging device for measuring an image profile of an object, particularly but not exclusively for measuring subcutaneous tumours.

### Background

Preclinical scientists and technicians are familiar and comfortable using hand-held calipers for tumour volume measurement (despite their current inaccuracy). Other tools based on imaging generally require workflow changes with either the eg animal subject being taken to an instrument, or an image to be captured of the mobile subject. Both approaches are currently expensive.

The use of callipers generally involves taking two distance measurements, eg along a short axis and a long axis, to then estimate the 3D volume. It is typically assumed that the short axis is equivalent to the height and that the tumour is a regular spheroid, so that an appropriate equation for that can be applied. However, tumours are often not regular spheroids, e.g. have lumps and bumps and can be flat or tall. This results in inaccuracy and variability in measurements. Variability is in particular high when two different people measure the same tumour, eg because they apply different amounts of pressure. Thus, calliper measurements can have low accuracy, high variability and a need for training of users. The variability may lead to lower confidence in the scientific result and may also mean that a higher number of animals (eg mice) are needed in a given research study to reach a statistically significant result.

In more detail, in the field of pre-clinical cancer research, estimating volume through multiple length measurements using tools such as callipers (whether manual or digital) are often restricted to two of the three dimensions because of accessibility issues where this third dimension is assumed to be equal to the shortest axis measured. Coupled with varying pressures applied on the tissue by different operators, this leads to significant variability of volume estimations, particularly between different users, in pre-clinical oncology studies. Where optical reconstruction of 3D surfaces is attempted, many external factors can influence the optical reconstruction of the measured surface such as ambient light, surface properties, hair/fur, as well as the subject not remaining still during the measurement.

It can be considered to use a tool with an array of pins to measure surface profiles, wherein each pin is connected physically to a microprocessor, to determine relative changes in z dimension. However, this can physically constrain the pin density and may come at a significant financial cost. Such a tool may also rely on locking the pins in place as well as the user providing downward pressure.

In this regard, it is noted that freely moving pins within an array are generally subject to lateral displacement when extended, hence the surface to be profiled generally needs to be contacted from the side or from the bottom. A subcutaneous tumour in laboratory mice, however, is more easily contacted from the top. In addition, the subject being a small living organism, only a certain level of pressure can be safely applied during the measurement.

For use in understanding the present invention, the following disclosures are referred to:

- WO9423605A1 (Crew et al) relating to a three-dimensional profile gauge;

- US6160264A (Rebiere et al) relating to plotting a tri-dimensional shape, notably a plantar arch, and method of operating the system for producing an orthopaedic shoe or sole;

- US2009112130A1 (Bengtson et al) relating to measurement and evaluation of tissue structures, such as breasts;

- WO2018/109453 A1 (Larkins et al) relating to obtaining data characterizing a three-dimensional object;

- "A pin-array method for capturing tissue deformation under defined pressure distributions and its application to prosthetic socket design" (Prince, Kenney & Howard. Medical Engineering and Physics, 84 (2020) 136 - 143).

Therefore, there remains a need in the field of surface profilers to improve speed, consistency, comfort, cost, convenience, accuracy, resolution and/or reliability of volume estimation of a profiled surface feature (eg subcutaneous tumour, for example of a mouse), the capturing of a greater proportion of the profile of the surface feature, a reduced need for user training to perform a measurement, a reduced number of subjects (eg mice and/or tumours) to achieve statistically different research results, etc.

## Summary

According to a first aspect of the present invention, there is provided an imaging device for measuring a profile of an object in accordance with claim 1.

This device can facilitate imaging of subcutaneous tumours in mice instead of using the conventional ultrasound scanner or micro-CT (MicroComputed tomography) scanners where the subject has to be tightly restrained or anesthetised. The conventional techniques are time consuming and expensive.

The moveable parts may be columns eg pins. Regardless, the parts preferably slide parallel to each other.

In a preferred embodiment, the device further comprises an objective lens between the moveable parts and the camera to create the telecentric system. (In embodiments, such an objective lens may, when placed at an appropriate distance, form a telecentric system with an objective lens of the camera). The telecentric system generally provides an orthographic projection, providing the same magnification at all distances, and therefore may in embodiments remove from an image of the moveable parts, eg columns such as pins, the height dependent change. This leads an improved performance due to reduced crosstalk that may be introduced to adjacent eg pins physically obstructing each other. In some implementations, the device further comprises a window between the telecentric system and the moveable parts, wherein the window is transparent to wavelengths of the light emitted by the optical source and the material. Such a window may be advantageous for use with a reset mechanism for the moveable parts, eg a pin reset mechanism.

In some implementations, the device further comprises a mechanism for resetting the positions of the moveable parts, the mechanism preferably comprising a spring and a switch attached to the window. The switch when activated may cause the spring to reset the positions of the moveable parts to a baseline. Other pin (or other type of moveable part) resetting mechanisms may include but not limited to blowing/forcing compressed air into the enclosure to set the position of the pins to a baseline, or using magnetic material attached to the end of the pins and then applying magnetic field to return the pins to the baseline.

In some implementations, the moveable parts comprise columns such as pins, the parts preferably formed of metal, plastic or fluorescent /phosphorescent material. The use of metal, plastic or fluorescent /phosphorescent material itself may be based on the cost and/or ease of manufacturing the imaging device. The parts are preferably loose enough so that the user is not putting so much pressure on a subject to be measured but not so loose that they fall out of the enclosure. Sideways movement of the parts, eg pins, may preferably be avoided to prevent them colliding with each other.

In some implementations, the enclosure comprises an array of holes corresponding to respective said parts (eg pins) such that the parts can move freely longitudinally along an axis of said enclosure. It is noted that in some embodiments the enclosure may not be solid, eg may be hollow, to reduce friction on the parts. This may be advantageous if the eg pins bend or lean sideways, for example when placed over an object with steep sides.

In some implementations, wherein the enclosure comprises a stop to restrict movement of the moveable parts out of the enclosure.

In some implementations, the fluorescent or phosphorescent material comprises a homogenous layer or coating, preferably sprayed or painted. However, the fluorescent or phosphorescent material may also be patterned, for example, comprising concentric rings or a cross.

In some implementations, wherein the first end of the moveable part contacting the object comprises a rotatable element, preferably a ball bearing. Using this technique allows better surface contact of the device with an object.

In some implementations, the first end of the moveable part of the imaging device contacting the object comprises a sensor operable to determine properties of the object to be measured, preferably applied pressure or temperature. In one embodiment it is possible to attach a sensor / mechanism to ensure that all users engage the profiler to the animal subject with comparable forces thereby reducing inter-operator variability. In one embodiment thermistors, thermocouples or resistance temperature detectors (RTDs) are attached to each moveable part, which may allow the measurement of surface temperature for each point within the array. This information can be used to assess fluctuations in temperature across the surface, allowing the identification of inflamed areas of joints.

In some implementations, the imaging device further comprising at least one spring attached to a respective said moveable part and the enclosure, the spring to enable determining the force generated to push the part to its respective position. Use of an imaging device in this manner allows to access tensile properties of the tissue under examination.

In some implementations, the imaging device further comprising a cap configured to provide a barrier between the moveable parts and the object, preferably wherein the cap is disposable.

In some implementations, the imaging device uses a monochrome camera. However, it is possible to use a colour camera with multiple channels which may provide an improved image. For example, if the fluorescent or phosphorescent material (eg paint) of an embodiment emits in the green range, extracting only a green channel from the image may improve a background/noise ratio and/or may reduce, eg prevent, chromatic aberrations that may affect focus of light of particular wavelengths.

According to a second aspect of the present invention, there is provided a method of modelling a profile of an object based on an image of moveable parts in contact with the object, the method comprising: (a) using a camera to obtain an image of illuminated ends of the moveable parts; (b) determining, for each said moveable part, a measure of blur; and (c) determining, for each said moveable part, a height or distance of the moveable part based on the determined measure of blur.

Preferably, the method comprises performing: a calibration procedure using a said object having a predetermined profile, wherein the calibration procedure comprises at least the steps (a) and (b) and generates a relationship between measure of blur and distance or height of at least one moveable part based on each determined measure of blur and the predetermined profile; and performing the method using an object having an unknown profile, wherein the step (c) is performed based on the generated relationship.

We further describe use of the imaging device or method to obtain a 3D image or model of removed tissue/tumour from a living organism.

We further describe use of an imaging device or method obtain a 3D image or model of a subcutaneous lump preferably of a human, for example where the lump is a cyst and/or is on a human neck.

We further describe use of an imaging device or method to identify and process objects comprising 3D QR code or Braille characters.

We further describe use of an imaging device and method where the sensor is a temperature sensor operable to measure surface temperature preferably of a human to identify inflamed areas of joints.

We further describe use of an imaging device on a tissue to access tensile properties of the tissue under examination.

However, uses of embodiments of the above aspects are not limited to the above applications.

The invention further provides processor control code to implement any of the above-described aspects, for example on a general purpose computer system or on a digital signal processor (DSP). The code may be provided on a carrier such as a disk, a microprocessor, CD- or DVD-ROM, programmed memory such as non-volatile memory (e.g. Flash) or read-only memory (Firmware). Code (and/or data) to implement embodiments of the invention may comprise source, object or executable code in a conventional programming language (interpreted or compiled) such as C, or assembly code. As the skilled person will appreciate such code and/or data may be distributed between a plurality of coupled components in communication with one another.

According to a related aspect of the invention, there is provided a non-transitory data carrier carrying processor control code which when running on a processor, causes the processor to implement the above-described method.

**Brief Description of the Drawings**

For a better understanding of the invention and to show how the same may be carried into effect, reference will now be made, by way of example, to the accompanying drawings, in which:

Fig. 1 shows an example in silico mechanism to test equivalent variable pin densities and variable measurements;

Fig. 2 shows example in silico volume estimations using various regular and irregular shapes;

Fig. 3 shows an example design of a pin-based mechanical profiler with optical processes for determining relative position of pins in three-dimensional space. An inlay of Figure 3B shows a phosphorescent sphere zoomed in for both a 0 mm and 12 mm image so the blurriness can be clearly observed;

Fig. 4 shows, for an example feature extraction algorithm, results for a single row of pixels at each stage in the pipeline for an image of a synthetic tumour;

Figure 5 shows, for example feature extraction algorithm, results for a single row of pixels at each stage in the pipeline for an image of a flat calibration surface;

Figure 6 shows an example assessment of measurement accuracy using defined 3D objects.

**Detailed Description**

Generally, we describe how a three-dimensional model of a surface is generated through placement of an array of moving parts, eg columns, over the object such that the profile of the object is captured in the profile of the parts. In embodiments, an image is taken of the tops of these eg columns or "pins" and this is used to estimate the distance that each column/pin has moved, eg relative to a predetermined position. This is then used to reconstruct the various heights (eg relative to predetermined positions), or distance from the ends of the pins to the camera, across the array, and therefore the object being measured, in three-dimensional space.

Embodiments allow a physical representation of the surface via an array of pins to be measured and quantified by capturing preferably (ie optionally) the entire (or substantially the entire) profile of a tumour surface. Heights or distance of pins may be inferred based on an image being taken from above the pin array through a telecentric system where, in contrast to a non-telecentric system, objects generally remain the same size regardless of their distance from the camera. Generally, the image of the end of a pin doesn't change as a function of height but the relative loss of focus does change. Thus, use of a telecentric system may mean that, as a pin moves towards the camera, the end of the pin appears 'blurrier'. A measure of 'blurriness', may represent the gradient of a transition of of the image intensity from a baseline or background level (this may be considered a 'no signal' level) to a feature intensity level (this may be considered) a 'signal' level, corresponding to a centre of a pin). Thus, edge enhancement for determination of blurriness may take the derivative, the Laplacian (i.e. second derivative), or other filters generally used for edge enhancement, of the image intensity profile to determine how steeply the intensity falls away at each pin, to where the edge of each pin is and how blurry its image is. A blurry image will have a more gradual transition compared to a sharp in-focus image. In this regard, it is noted that blurring happens in both directions, ie, both when the pins are closer to and more distant to the camera, for example depending on convex or concave surface variations. Using a set of objects with known but variable heights allows each pin to be calibrated based on the extent of 'blurriness' observed. The array may then be placed over the subject to be measured, an image captured, and heights / distances inferred using the calibration data. As each pin is part of a physical array, the approximated heights of each pin can then be used to reconstruct the three-dimensional surface of the object.

(We note that the present application generally uses the term 'blurriness'. However, this may be replaced with eg 'measure of blur', 'degree of blur' or, conversely, 'degree' or 'measure' of 'focus').

Generally, the tool is configured to have a baseline position of the pins such that blurring of the image only occurs due to movement of pins in a single direction. This may be achieved based on the geometry of the optics including at least the focal length (eg 78mm). The baseline position may be set based on a desired offset of eg 1mm, so that the movement generally cannot go towards the 'wrong' side of the focal point. The base position may for example be about 1mm away from position of the sharpest image, so that images will change to be progressively more blurred as the pins get closer to the camera and mechanically the pins cannot go further out. A calibration step prior to image capture may provide a reference for each set of one or more measurements against a surface, e.g., a single calibration image used for a set of 100 images. A calibration curve may be provided for each pin, wherein each pin may have a slightly different curve. Thus, the calibration step may generate a calibration curve for each pin.

Embodiments may be calibrated based on eg 1mm increments. This may correspond to the resolution of height / distance measurement of the device.

It is further noted that use of the telecentric system may mean that one pin will not obstruct imaging another pin. With a non-telecentric system, if one pin is high and thus has a larger image, it may physically obstruct imaging of an adjacent pin. With the telecentric system, an actual object doesn't appear larger or smaller based on distance but does become more or less out of focus. So with an algorithm, each pin position is relatively fixed but the blurriness as can be seen in the example of Figure 3B after the device is placed over a completely flat surface (0 mm) and a flat object with a known height (12 mm) where the image changes from being more in focus to more blurry when closer to the lens.

In silico testing identified preferred densities of pins and also confirmed that the approach may be reproducible for example even if the centre of the object is modified with respect to the pin array, simulating different users, and that the approach may avoid optical interference. Embodiments may allow rapid and/or reliable volume estimation of subcutaneous tumours.

Additionally or alternatively to the use of a telecentric system, other embodiments may use other features of the pin array image such as shape, size and/or intensity.

To assist with the capturing of an image, a number of strategies can be employed regarding illumination and/or other energy sources, detection, light path and/or positioning. The inventors have achieved good results by utilising the properties of phosphorescent and/or fluorescent molecules covering particles (eg spheres or beads for example 1mm diameter though more preferably flat particles) attached to the end of the pins. (The optional use of the particles may enhance consistency of coating of the phosphorescent/fluorescent paint compared to direct application of the paint to preferably flat pin ends. Alternatively, however the pins may be formed of phosphorescent or fluorescent materials so that no paint is required). The activation of the molecules by a light source followed by subsequent capture of an image led to low background images with no optical aberrations due to internal reflections that were found with other illumination/detection methods. (The use of phosphorescence or fluorescence, instead of illuminating the end of the pins with an optical source and measuring reflected light from the pins, generally reduces reflections from internal features of the tool, eg. from the lens, affecting the image). The molecules may be applied to the ends of the pins by mixing with glue and painting (eg by brush), or by spraying an aerosol type paint having the molecules. The molecules may be applied to elements such as spheres, that are attached (eg glued) to the pin ends before or after the painting.

In this regard, it is noted that the image and thus measurements obtained by an embodiment may depend on the loading (eg concentration and/or surface area) of the phosphorescence material and the intensity of light that you use to excite that molecule.

Multiple coatings of the material may be applied to increase the concentration and thus light emission from the pin ends.

In embodiments, a container preferably offers enough resistance to hold the pins in place without significant lateral movement and/or without it being difficult to return to baseline using a return mechanism such as physically pushing the pins back down using a springloaded platform within the device itself. This may be desirable regarding contacting the surface to be profiled, e.g., subcutaneous tumour in a laboratory mouse, from the top and/or with lower pressure applied to the surface.

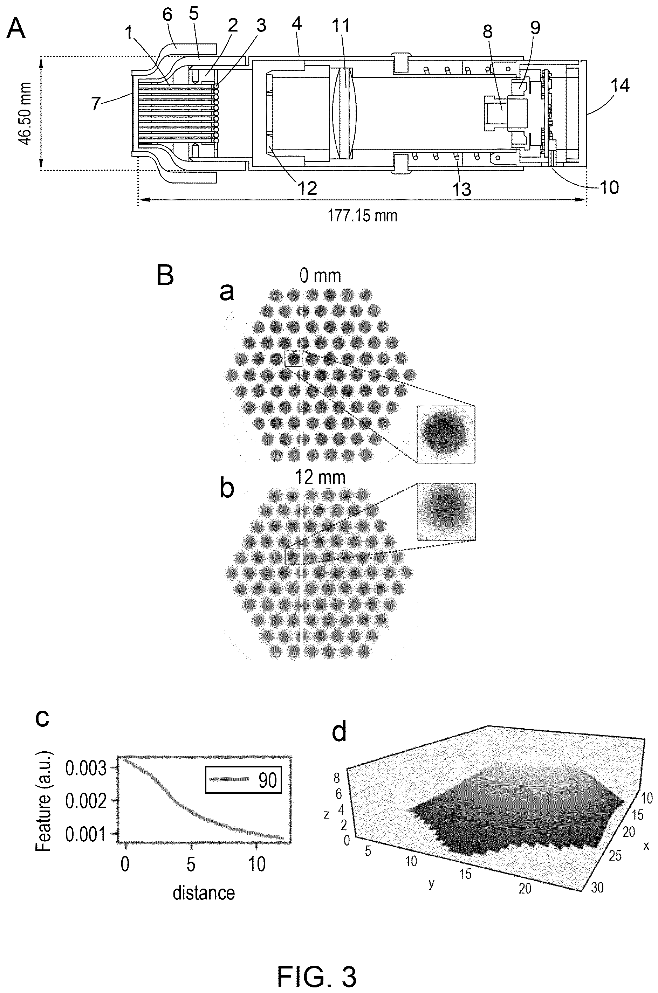

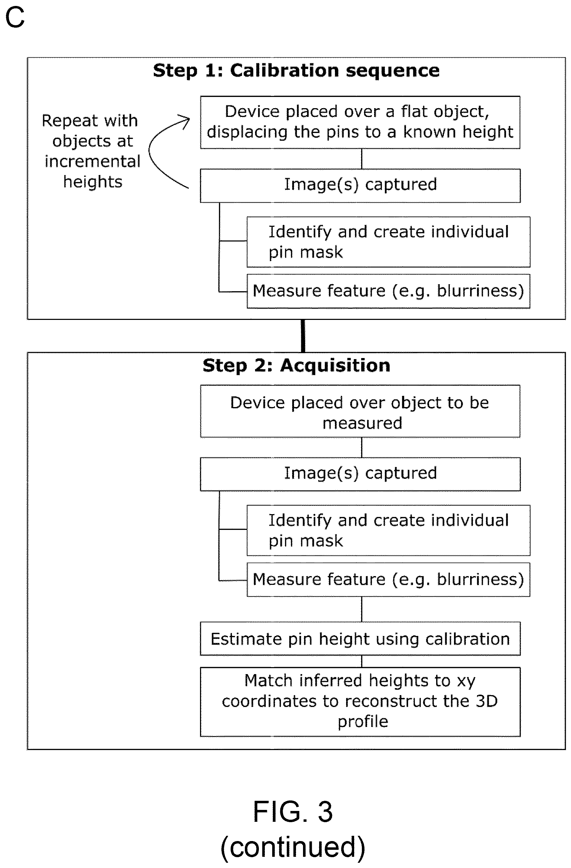

In this regard, it is desirable that the travel of the pins is as linear as possible, to avoid them going off axis and interfering with other pins. Preferably, resistance to movement of the pins is low, so that the surface to be measured i.e. an object is not crushed. However, the pins are preferably not so loose that excessive movement occurs in that they fall out of the block. Pin materials with appropriate coefficients of friction may be selected. For a home position the pins are sticking out as far as possible before the next measurement is taken. A preferred embodiment has tolerances to allow a sliding pin arrangement such that the pins fall down with ease, for example with percussive assistance (eg if you tap the tool) then the pins all fall down to their home position. Preferably, there is sufficient friction to resist the weight of each pin. However, even over time there could be debris and there could be wear. In addition, a mechanism may be provided to prevent the pins sliding back towards the camera in view of the potential that, if the device is rotated beyond horizontal. In this regard, Figure 3 shows a flat element 12, wherein the mechanism is spring loaded by a spring 13. When a user pushes on a button 14, they may then push at least the part of the internal assembly forwards and to return the pins to their home position. The flat element 12 is preferably transparent, eg a glass window. Being provided to push the pins back to their original positions, such a transparent element may nevertheless in embodiments be helpful to allow imaging of the phosphorescence, i.e., allow pushing the pins back to their original position without optically blocking the measurement.

Advantageously, no direct electrical or other connection to each pin may be needed to measure changes in pin height, for example because in embodiments the ends of the freely moving pins are imaged using a camera. Combining this with optional measurement of additional metrics such as surface temperature and/or including springs (or other sensors in the pins to measure other biochemical / physiological properties of the underlying tissue) on the pins with known tensile properties may provide information in addition to the inferred 3D profile.

The external ends of the pins may be shaped to allow good contact with the surface to be profiled. The ends may be flat or rounded. A rotatable element such as a ball bearing may be provided at each of the ends, so that when the tool is moved over the surface it can glide rather than potentially catching eg on skin. This may allow the tool to continuously cover a large surface and/or better capture contours of the surface. Such an embodiment may be coupled with a gyroscope and/or positioning monitoring system, to allow determination of the device's orientation and/or position during surface image capturing. Such information may allow reconstruction of a large surface.

In a preferred embodiment, the measurement acquisition is initiated by a non-manual means (e.g. footswitch or voice) to permit the preclinical scientist / technician to maintain the animal subject-to-profiler orientation in a stable and secure configuration.

In a preferred embodiment, the system integrates a "live mode" in which a sequence of images is rapidly captured (preferably as close to real-time as is possible) to permit improved placement of the profiler over the underlying tissue.

Figure 3A shows an embodiment having an array of columns or "pins" (1). In this regard, the inventors have found that across a certain area a minimal number of contact points is preferred for good measurement accuracy and easier to manufacture. In embodiments, that may be eg about 50 - 100 pins over a 2 cm squared area.

The following generally concerns the mechanical design and overall process design. From 'in silico' data described below, the inventors concluded an array of approximately 90-100 pins would allow accurate reconstruction of three dimensional objects. A system built on these specifications is described below.

Figure 3A describes one embodiment containing an array of columns or "pins" of eg 1.5 mm diameter (1), likely being made of metal or plastic (polymeric may provide advantages regarding eg shape, form and/or cost). The pins are placed through a block (2) containing a corresponding array of holes such that they have sufficient freedom to move up and down. The shape of the pins and/or addition of a fixed stopper hinders or prevents them from falling out from the either side. In the current embodiment, a plastic sphere (3), spray-painted with three coats of phosphorescent material (eg approximately 25-30 microns thick) that emits in the green (eg ~550 nm), is fixed on the camera-facing end of each of the pins. In the current embodiment this includes an even distribution of phosphorescent material across the sphere but could also be across other shapes or in various patterns (e.g. cross or concentric circles). The phosphorescent material emits green light after exposure to a light source such that the timing of image acquisition can be suitably delayed such that internal illumination/reflections of the undesired aspects of the assembly can be eliminated.

The block (2) is attached to external housing tube (4) such that one end of the pin array can contact the surface to be measured and the other end is enclosed by the housing. A shroud (5) covers the pin array protecting the pin assembly and can be designed to be different lengths to expose different lengths of the pin array should it be required. An additional cap (6) containing a flexible membrane (7) is pressed onto or locked over the end of the device, presenting a protective barrier between the object and the device as well as a more suitable surface to interact with the pin array. Contained at the end of the housing is a camera (8) with a traditional 8 mm lens and a blue (445nm wavelength) LED light source (9) with associated microelectronics that is at a specific distance away from the pin array when in its lowest position. In the current embodiment, the camera (8) is a CMOS monochrome, 5-megapixel camera. However, use of a colour and/or higher resolution camera in future embodiments could allow improved image analysis, for example, specifically using the green channel and detecting blurriness more reliably, respectively. A cable (10) connects the camera to a computer system (not shown in the diagram). When a measurement is performed, the light source (eg LED) is turned on and off to excite the molecules immediately before the imaging.

The cap (in embodiments disposable) may be an external, detachable (eg clip-on/off) part. It may provide a membrane across the surface, preferably to be attached and then detached after a measurement. This may provide flexible membrane material as barrier between the surface and the pins. The cap may serve multiple purposes such as reducing ingress of fur and dust into the mechanism, providing a sterile protective barrier between the subject and the device to reduce transfer of pathogens (eg between mice), and/or reducing the effect of surface stickiness movement of the pins. Thus, when the cap is attached, the pins may have improved movement.

In between the camera and the pin array is an objective lens (11), which, in combination of the camera lens (8), spaced apart by the focal length of the objective lens (11), thereby forming a telecentric system, causes captured images of objects to appear sharp or blurry when moved axially towards/away from the camera (8) rather than a traditional lens where objects appear bigger or smaller when moved closer or farther from the camera. Using a non-telecentric system, a pin may obstruct neighbouring pins when at its highest position, whereas a telecentric lens may generally avoid this issue. In the current embodiment, the objective lens (11) is a plastic aspheric lens 35 mm in diameter with a 53 mm focal length. Another embodiment omits the objective lens and/or the phosphorescent paint such that the ends of the pins can be imaged and pin height inferred by relative size of each pin end, which is relative to distance from the camera. Between the lens and the pins is a clear disc (12) connected via a large spring (13) to a button or lever (14) at the end of the housing which pushes the pins back to the lowest position when the button or lever is depressed.

To demonstrate the change in "blurriness" as the pins are moved closer towards the camera, the device may for example be placed over a completely flat surface (0 mm) and a flat object with a known height (12 mm) leading to discrete changes in pin height and therefore "blurriness" (Figure 3B, top two panels). When the device is placed over a series of calibration objects at incremental heights (e.g. 1 mm steps) and a numerical value for "blurriness" is obtained, this can be displayed as a line graph showing the correlation for each pin between the numerical feature and distance. When the distance is computed for each pin individually, the entire array can be reconstituted using it's known x and y coordinates as well as it's interpolated z coordinate (Figure 3B, bottom two panels). The proposed preferred sequence is demonstrated in Figure 3C which comprises a calibration step with the device being placed over the series of calibration pieces to generate a standard curve for each pin with respect to blurriness and distance, and a subsequent acquisition step with the device being placed over the object to be measured.

However, the design of the embodiment may vary for example with respect to one of more of:

- pins (eg pin number, arrangement, shape and/or material);
- method for measuring pin heights: eg standard imaging (size of object = distance to camera), multiple images taken at multiple points in any dimension (x,y,z), distortion of physical or projected image;
- assembly: eg contained within one solid unit or as separate units where the pins are locked in place, having extruding or covered pins;
- optics: eg containing mirrors or filters, containing additional light/energy sources, additional detectors; and/or
- processing: eg using different pin features or variations thereof instead of "blurriness", using specific channels, cropping, or altering image prior to processing, using machine learning/AI to identify features.

Regarding processing of the camera output image, a preferred method involves use of a 'blurriness' measurement as a parameter to make a height or distance estimation. An embodiment may first create calibration metrics for example comprising as a range of known 'blurriness' values and known heights / distances. These may be used to determine where each pin is positioned in real space based on an image of the back ends of the pins. The determination may determine precisely where the centre of the pin is within the physical array. From that the heights / distances (z) are computed as well as the x y positioning and then from an interpolation a reconstructed 3D volume or 3D image eg of a tumour can be computed as the final metric. The interpolation may in effect use a 3D graph based on the computations of the x y values and the blurriness, the graph comprising lines fitted to interconnect points in space to allow a volume to be computed.

The blurriness-based method may be used regardless of whether phosphorescent / fluorescent molecules are used, eg may be applied if reflectance of light from the source by the pin ends is used. However, the telecentric system is present. The molecules may however be advantageous for reducing noise, in embodiments allowing capture of just the emitted light ie not including light that is internally reflecting around the inside of the device. Thus, the signal to noise ratio of the image may be improved, leading to a cleaner image as an input to the algorithm.

The following describes in more detail an algorithm that determines distance or height of pin(s). The algorithm generally aims to generate a blurriness measure and correlate with distance or height. In embodiments, the blurriness measure for each pin may be determined by detecting (eg using edge detection processing) any area(s) of high degree or rate of change (eg gradient or curvature) of intensity within an image region that may be associated with the pin. In embodiments, the sum of the degrees/rates across the measured local region may identify the maximum blurriness for each pin as well as its central position.

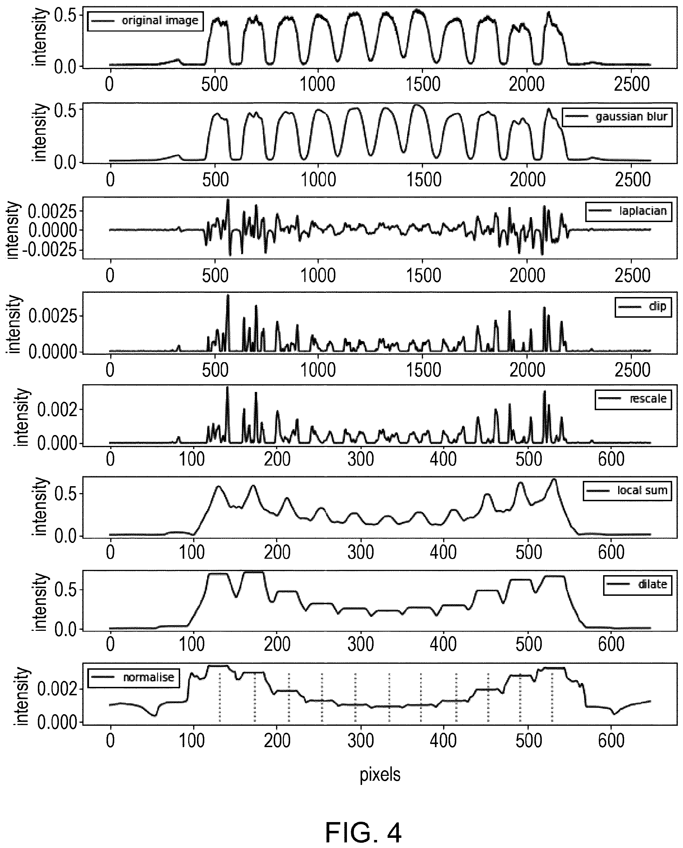

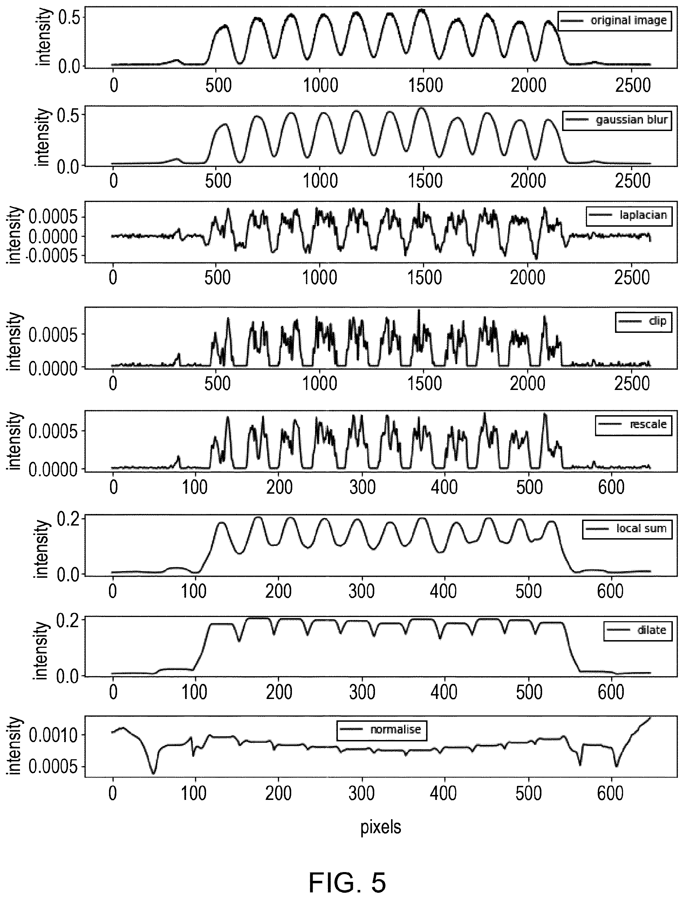

Thus, for some embodiments, for each pixel the intensity change values (eg curvature) measured in a local region (eg 10 or 16 pixels) around that pixel are summed together such that when that local region contains all of the pixels that come from a pin the value is at its highest. This corresponds to for example the peaks seen in the 6th panel within Figs. 4 and 5 and is a "blurriness" measure (in embodiments, the final reported version of the measure). It can be seen in the graphs that the peaks go from ~0.0007 to 0.2 on the y axis after the sum step as a result of all the local region around a pixel having been added together.

To further understand the summing, it is noted that in embodiments a broad step preceding the summing may quantify a measure of the degree or rate of intensity change at each pixel (preferably, intensity curvature) where instead of a luminance value for each pixel we now have the eg "degree or rate of change" value for each pixel based on a eg Laplacian step. After clipping these values to a positive value, the summing step may involve, for each pixel, summing this clipped value across a preferably disk-shaped region that has a radius of a predetermined number, eg 16, pixels so that, as the pixel being computed moves closer to the centre of the pin in the image, the highest value may be obtained when this summed area covers most/all of the pixels associated with the pin. Preferably, there is no selecting or removing of any low degree/rate (eg curvature) pixels in the summing step; the image region is assessed as a whole.

(Regarding any such clipping, it is preferred to sum all the positive values. Preferably, the absolute values are summed if an embodiment uses a 2nd derivative, otherwise there may be a risk of everything cancelling out in the summing operation (note the positive and negative peaks of roughly the same size in subplot 3 of Fig. 4). Generally, an edge always generates both a positive and negative spike in the Laplacian).

The detection of a 'high' eg curvature may be relative to a threshold value. As suggested above, a preferred embodiment may perform the detection of the sub-region(s) of high curvature by using a Laplacian filter. The summing may be performed using for example a top hat filter. A dilation process may be performed so that it doesn't matter if the pins are in a slightly different place in any calibration image(s) relative to the measurement images.

In this regard, it is noted that blurry images generally can't change intensity very quickly. An embodiment may therefore first filter the image to emphasise region where the intensity is changing quickly. There are a variety of options for doing this, for example a Laplacian, bearing in mind that looking at the second derivative may give a response that is more different between a sharp edge and blurry edge. However, other operations may work just as well e.g. absolute gradient, higher derivatives, or other common edge detection filters.

Where the method for detecting sharp edges is a second derivative, eg using a Laplacian as mentioned above, this may also emphasise any random noise in the image. Noise filtering, eg using a Gaussian filter, may therefore be applied to the image as a first step to smooth out any noise before proceeding with the eg Lapacian. However, such noise filtering may be less advantageous if uniform pin images are captured by the camera. Thus, a degree of Gaussian filtering may be reduced or not used at all, which may turn give more sensitive edge detection, particularly close to the in-focus point.

Consistent with the comments above, having created an image with the 'regions of high change' emphasised, embodiments may then sum all the regions of high change associated with a particular pin. It is noted that there are however lots of ways to do this. For example, the algorithm could run a blob detection algorithm and then use the centres of the blobs as the centres of the pins and sum all the pixels around the estimated pin centre. The algorithm may then correlate the pin centres from the measurement image to the pin centres in a calibration image to find the most likely match and pick the most appropriate calibration curve. However, a computationally faster approach may be to use a top hat filter to sum the pixels around every pixel. At every point the top-hat filtered image may contain the sum of the surrounding pixels in the edge emphasised image. The points of highest intensity in this image will generally be ones lying near the centre of the pins because at these points the top-hat filter will be overlapping with, and therefore summing, the most points with high change.

A further, sometimes final, step is to perform a dilation which, in essence, smears out the peaks from the summing step. So, instead of explicitly matching pin locations from the calibration image to pin locations in the measurement image, the algorithm may simply take the pin location from the calibration data and look at that exact location in the dilated measurement image. Because the dilation step has been performed, it doesn't matter that the algorithm is not looking in quite the right place.

The following generally concerns the 'in silico' data referred to above.

To test the potential of an array of measurements to estimate object volume, a pipeline was generated using ImageJ utilising an iterative resize:scale method to simulate array resolution.

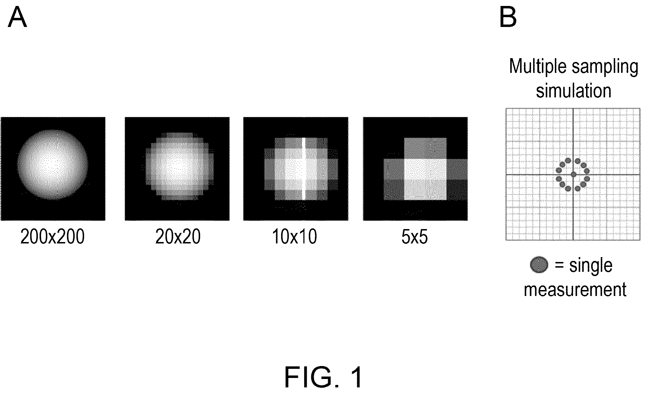

Grayscale shapes from 3D objects were used where the intensity gradient correlated to the object height (z dimension). (In embodiments, the absolute intensity, rather than intensity gradient (eg differential), correlates (eg linearly) to height. In this in silico modelling example, test pixel brightness is thus used as a surrogate for height determined by blurriness). An array area of 2 cm by 2 cm was used in a 200 pixel by 200 pixel array, giving 10 pixels/mm. Shapes were constructed in Photoshop, saved as tiff images and processed within ImageJ. Images were re-sized to 20x20, 10x10 & 5x5 pixel arrays without averaging or interpolation and then scaled back up to a 200x200 pixel image (again without interpolation) to mimic a lower resolution of pins (i.e 1 pixel = 1 pin, Figure 1A). Using the average intensity, area, known height and maximum intensity the in silico volume could be estimated using the equation below:

$$\text{Volume (mm}^3\text{)} = (\text{area} \times \text{mean Intensity}) \times (\text{known height/maximum intensity})$$

Given a standard area of 400 mm$^2$, the height of the object = 1 cm and maximal intensity of grayscale pixels = 255, the volume could be estimated. The centre point of the object in the image was translated radially around the original centre point to mimic placement of the measurement array at different positions (i.e. inter/intra-operator variability, centre positions depicted in Figure 1B) and the object intensity measured. This was compared with length measurements equivalent to callipers where "short" and "long" distances were measured and used to estimate volume using the equations below:

- Volume (mm$^3$) = $\pi/(6a^2b)$ - where $a$ = "short" axis and $b$ = "long" axis and the object height is assumed to be equal to a as with current calliper measurements; or

- Volume (mm$^3$) = $\pi/(6abc)$ - where $a$ = "short" axis and $b$ = "long" axis and $c$ = height set at 1 where object height is manually entered as a comparison with the above equation

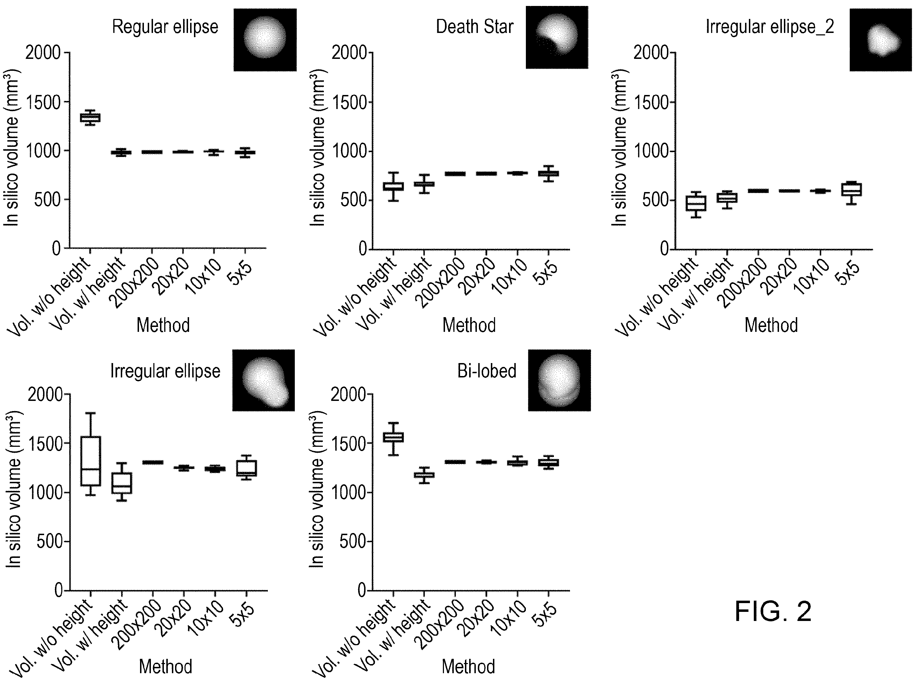

Multiple "short" and "long" measurements were taken around what could be considered reasonable points of measurement.
The baseline comparison in this test used two distance measurements as with calliper measurements where the height of the object is not measured. The 200x200 array gave identical repeated measurements and was the most accurate measurement method. Practically, however, an array of 40,000 pins is not possible. Interestingly, reducing the array size to 20x20 or 10x10 was still highly accurate with minimal variability. A 5x5 array gave the highest variability but was still similar to distance measurements with known heights, and still superior to distance measurements where the height is estimated. Overall, the measurement array was superior across multiple shape types and a 10x10 array should be sufficient to maintain accuracy and reproducibility as shown in Figure 2. Distance-based assessment of volume, as used with calipers, can sometimes accurately estimate tumour volume of a regular ellipse when the height is known but generally overestimates volume if the third dimension is assumed (Figure 2, top left panel, first two columns). However, the ability to accurately estimate irregular shapes that contain gaps, additional lumps or lobes is compromised using distance-based methods, regardless of whether the height is a known or assumed (Figure 2, remaining four panels). A measurement array, however, was able to estimate volumes with minimal error across all the shapes tested.
The following generally concerns a tumour 3D profile reconstruction algorithm. In overview of an example (wherein any one or more of the steps may be omitted):

1. For each pin compute a measure of 'blurriness'. Create a calibration curve mapping 'blurriness' to distance.

2. For each pin work out the transformation from pixel space to 'real' space.

3. For each pin use the calibration curve and the pixel space to real space transformation to compute z and x,y co-ordinates respectively.

4. Perform an interpolation, e.g., linear interpolation, between pin x,y,z co-ordinates and then integrate to get the final volume.

We further provide an overview of an example computation of a 'blurriness' measure by detecting regions of high intensity curvature and summing them together over the region of the pin (wherein one or more of the steps that may be more preferably omitted are denoted by an asterisk):

1. **Gaussian blur** (to reduce sensitivity to noise)*

2. **Laplacian filter** to highlight regions of the image with strong edges

9

3. **Clip** to positive values to focus on the outer edges of the pins where the intensity curvature is positive.

4. **Local sum** via convolution with circular top hat filter. Essentially summing all the regions of positive curvature around a pin.

5. **Dilate** - Perform morphological dilation[1] (smearing out the peaks so it doesn't matter if we don't know exactly where the pins are) - call the result of steps 1-5 image A. (As noted on https://en.wikipedia.org/wiki/Dilation_(morphology), Dilation - usually represented by ⊕ - is one of the basic operations in mathematical morphology. The dilation operation usually uses a structuring element for probing and expanding the shapes contained in the input image.)*

6. **Sum raw image without Laplacian filter** - Return to the original image and perform the same gaussian blur as step 1, followed by the filter in step 4 and then the dilation in step 5 - call the result image B*

7. **Normalise** - Divide image A by image B and call this image C. This step is intended to normalise for changes in illumination level.*

8. **Look-up blurriness measure** - Look up the pixel values in image C at the expected location of the pins. The pixel value at these locations is our 'blurriness measure' for each pin.

Figure 4 shows the results for a single line of pixels across the centre of the array at each stage in the algorithm. The final values of the metric are found by looking in the image at the expected location of the pins. The expected locations are determined from the calibration images and are shown by the dotted lines in the final subplot. The value of the blurriness measure is the intersection between the dotted lines and the solid line in the bottom subplot. The flat regions in this plot may help to ensure that there is substantial tolerance to changes in pin position relative to the calibration sequence.

Figure 5 shows, for example feature extraction algorithm, results for a single row of pixels at each stage in the pipeline for an image of a flat calibration surface.

To perform step 8 in the algorithm above it is desirable to know where to find the pin centres. An example algorithm for this is:

1. Collect N calibration images from calibration pieces having flat surfaces.

2. Sum N calibration images to form a mean image.

3. Perform blob detection using the 'laplacian of gaussian' method (https://scikit-image.org/docs/dev/api/skimage.feature.html#skimage.feature.blob_log)

4. Use the blob centres as the pin centres.

To be able to reconstruct the 3D volume, it is desirable to know where the pins are in 'real' space rather than pixel space. The following is an overview of an example conversion of pixel space to real space:

1. Compute the image pin centre locations as above

2. Load expected pin locations in 'real space' from CAD data

3. Run a global optimiser to compute the rotation, scaling and translation that aims to optimally map image space to real space.

4. Discard the rotation and translation but retain the scaling for use in the 3D reconstruction.

Alternatively, if part to part mechanical variation is low then this conversion can be omitted and a fixed value for scaling is used instead.

For reconstructing 3D pin locations, points for 3D reconstruction may be computed by the following example approach:

1. Calculating z values by computing the blurriness measure described above and then comparing to a calibration curve;

2. Calculating x,y values for each pin by applying the pixel space to real space transformation.

Finally the 3D volume may be computed via a linear interpolation between each x,y,z coordinate for each pin.

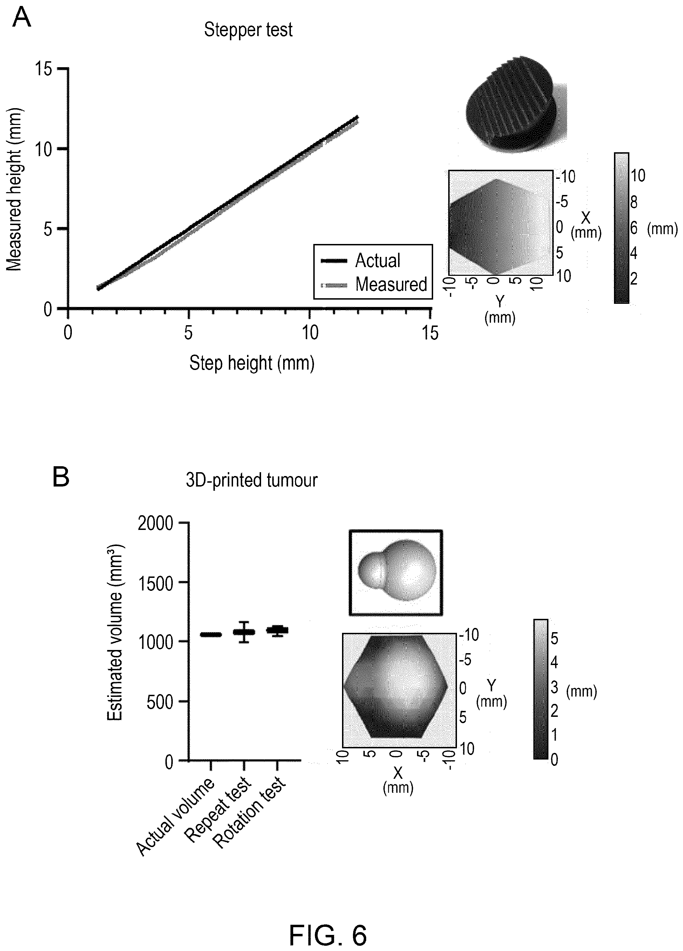

The inventors have further considered accuracy and repeatability testing. To test the accuracy of the system in measuring and reconstructing three-dimensional geometries the inventors placed the device over distinct shapes with known dimensions. The first object contained 10 "steps", each approximately 1.2 mm high and ranging from 1.2 to 12 mm in height (Figure 6A). A calibration image set was captured before placing the device over the object, capturing the image and computing the heights of each pin. The average height detected for each "step" was plotted against the known height of each "step" in an xy graph (Figure 6A). The second object was a bi-lobed, half spheroid generated using 3D printed plastic with a known volume of 1059.6 mm$^3$. The object was measured using the device with repeated measurements (eight independent off/on interactions) and rotational measurements (eight independent interactions at iterations of sequential 45° angle movements between images) and results were described (Figure 6B). The statistics are further described in Table 1.

**Table 1**: Initial performance testing statistics

|  | Repeat test | Rotation test |
|---|---|---|
| **Number of values** | 8 | 8 |
|  | Units: mm$^3$ | Units: mm$^3$ |
| **Minimum** | 992.0 | 1043 |
| **Maximum** | 1165 | 1126 |
| **Range** | 173.0 | 83.00 |
|  |  |  |
| **Mean** | 1081 | 1093 |
| **Std. Deviation** | 49.84 | 30.10 |
| **Std. Error of Mean** | 17.62 | 10.64 |

Regarding use cases, embodiments may generally be used for clinical evaluation of biological surfaces and materials, and/or to analyse three dimensional structures designed to store data. Embodiments are discussed herein merely in relation to tumours such as a subcutaneous tumour (generally involving a skin-based profile measurement) or a removed tumour. However, embodiments may be applicable to measurement of other human / animal tissues, and/or to non-animal/human surfaces, such as surface of plants or other inanimate objects.

More specifically, applications outside pre-clinical oncology may include other medical applications such as measuring joint inflammation as part of rheumatological assessment as well as general external surface assessment such as palpable lumps of the skin (eg neck lumps) etc. It is also to be noted that embodiments may have utility as a way of decoding information stored in 3D geometries in a similar way to QR codes but using multiple points along the z axis to define a variable. For example, standard QR codes use black or white boxes arranged in patterns that correspond to information such as binary sequences. A 3D QR Code may however be considered. By each box having multiple Z positions, the amount of information that can be stored in each "box" is greater.

Example technical details of clinical evaluation of biological surfaces and materials are described below:

- Surface profiles of human tissues accessible from the exterior, such as skin, skin-based growths or tumours, bone, joints and bony processes or lesions, or profiles of tissues removed after surgery (i.e. *ex vivo*) can be generated using the current embodiment of the technology using the method described. This can be used to document basic information regarding the shape and size of the measured tissue that could aid clinical evaluation of the tissue. With respect to *ex vivo* tissues, a plurality of pin arrays could be used to assess the entirety of the tissue surface.

- Further information can be acquired by integrating existing technology into the device. For assessing tensile properties of the tissue under examination, springs with a known spring force can be attached between the pins (1) and the block (2) such that the extent of pin movement will be directly proportional to force applied. When sequential images of the pin heights are obtained during application, the extrapolated force applied can be measured at each stage. Furthermore, addition of thermistors, thermocouples, or resistance temperature detectors (RTDs) to each pin (1) allows the measurement of surface temperature for each point within the array. This information can be used to assess fluctuations in temperature across the surface, aiding identification of key areas of interest (e.g. inflamed areas of joints).

Thus, for example, measuring joint inflammation relevant for arthritis monitoring may be achieved by embodiments.

Example technical details of methods to analyse data-encoded 3D structures are now described. Embedding coded information within a visual format has been utilized successfully with barcodes and "quick response" (QR) codes. Typical QR codes use black and white blocks to represent binary code in blocks of eight to code for specific characters. Contrary to previous iterations of physical codes detected by a 2-dimensional image sensor, a 3-dimensional profile can be captured as previously demonstrated and then analysed digitally using a programmed processor. In the current embodiment, each pin could be used to distinguish, for example, 128 distinct positions per pin subsequently used to encode information within each pin. Using, for example, a general purpose 7-bit encoding method would allow data such as URLs to be encoded within a 3-dimensional array of columns. Further information such as orientation, error-correction and encoding format could be included by using the physical layout, shape or colour of the pins.

Custom storage and encoding of data could also be included. For example, using 40 distinct positions could capture information using a "tetracontadecimal" system where each position corresponds to 40 numeric characters and a group of three pins read in a sequence could represent numbers up to 65,640. Using a customised encoding format, these numbers can be used to encode for whole words, word families, unique characters or sequences. Applications could include identification/monitoring of equipment or products that have a manufactured "3D" QR code as well as transfer of information within physically interactive points of interest such as museums or exhibits, with potential to be interfaced with Braille technology.

It is noted that the use of blur is a feature of a number of embodiments as described above. In any of these embodiments, the blur may generally be calculated using a series of chained image processing techniques which detect ends of the moveable parts (e.g., pins), preferably remove image artefacts/noise, and extract and measure features of interest, such as local extrema, gradients and/or intensities, etc. These feature(s) or measurement(s) may then be used alone or any combination as, or to determine, a measurement for blur.

No doubt many other effective alternatives will occur to the skilled person. It will be understood that the invention is not limited to the described embodiments and encompasses modifications apparent to those skilled in the art lying within the scope of the claims appended hereto.

**Claims**

1. An imaging device for measuring a profile of an object, the device comprising:

   a plurality of moveable parts (1), wherein each moveable part has a first end to contact an object and a second end comprising fluorescent or phosphorescent material;
   an enclosure to hold the moveable parts such that the moveable parts are configured to move parallel to each other within the enclosure to vary their respective positions according to a profile of the contacted object;
   a light source (9) to stimulate light emission from the material;
   a camera (8) configured to obtain an image of the second ends of the moveable parts based on the light emission; and **characterized in that** the device further comprises
   a telecentric system between the moveable parts and the camera (8).

2. An imaging device of claim 1, comprising a window between the telecentric system and the moveable parts (1), wherein the window is transparent to wavelengths of the light emitted by the optical source and the material.

3. An imaging device of any preceding claim, having a mechanism for resetting the positions of the moveable parts, the mechanism comprising a spring (13) and a switch attached to the window, the switch configured to, when activated, cause the spring to reset the positions.

4. An imaging device of any preceding claim, wherein:

   the moveable parts (1) comprise columns such as pins, the parts preferably formed of metal, plastic or fluorescent /phosphorescent material; and/or
   the first end of the moveable part contacting the object comprises a rotatable element, preferably a ball bearing.

5. An imaging device of any preceding claim, wherein:
   the enclosure comprises:

an array of holes corresponding to respective said moveable parts (1) such that the moveable parts can move freely longitudinally along an axis of said enclosure; and/or

a stop to restrict movement of the moveable parts out of the enclosure, and/or wherein the imaging device comprises a cap configured to provide a barrier between the moveable parts and the object, preferably wherein the cap is disposable.

6. An imaging device of any preceding claim, wherein the fluorescent or phosphorescent material comprises a homogenous layer or coating, preferably sprayed or painted.

7. An imaging device of any preceding claim, wherein the first end of the moveable part (1) contacting the object comprises a sensor operable to determine properties of the object to be measured, preferably applied pressure or temperature.

8. An imaging device of any preceding claim, comprising at least one spring (13) attached to a respective said moveable part and the enclosure, the spring configured to enable determining the force generated to push the moveable part to its respective position.

9. An imaging device of any preceding claim, wherein the camera (8) is a monochrome camera (8).

10. An imaging system comprising the imaging device of any preceding claim, the system configured to, for each moveable part, part (1), determine based on the image a measure of blur and determine a distance or height of the moveable part based on the determination.

11. Use of an imaging device of at least claim 7 of the preceding claims, wherein the sensor is a temperature sensor operable to measure surface temperature preferably of a human to identify inflamed areas of joints.

12. Use of an imaging device of claim 8 or any claim dependent thereon of the preceding claims on a tissue to assess tensile properties of the tissue under examination, wherein the tissue is of a said object, the properties comprising a tissue property that determines a force generated by the tissue to push a said moveable part to its respective position, preferably wherein the imaging device is according to claim 8 and the generated force is enabled to be measured using a said spring.

13. A method of modelling a profile of an object, the method using the imaging device of any one of claims 1 to 9, wherein the image is based on an image of moveable parts constrained to move parallel to each other, wherein the image is of the moveable parts in contact with the object, the method comprising:

(a) using the camera (8) to obtain an image of illuminated ends of the moveable parts;
(b) determining, for each said moveable part, a measure of blur;
(c) determining, for each said moveable part, a height or distance of the moveable part based on the determined measure of blur.

14. The method of claim 13, comprising performing:

a calibration procedure using a said object having a predetermined profile, wherein the calibration procedure comprises at least the steps (a) and (b) and generates a relationship between measure of blur and distance or height of at least one moveable part based on each determined measure of blur and the predetermined profile; and
performing the method using an object having an unknown profile, wherein the step (c) is performed based on the generated relationship.

15. The method of claim 13 further comprising obtaining a 3D image or model of any one of:

a subcutaneous tumour;
removed tissue/tumour from a living organism; or
a subcutaneous lump preferably of a human, for example wherein the lump is a cyst and/or is on a human neck, or to identify and process objects comprising 3D QR code or Braille characters.

**Patentansprüche**

1. Bildgebungsvorrichtung zum Messen eines Profils eines Objekts, die Vorrichtung umfassend:
   eine Vielzahl beweglicher Teile (1),

   wobei jedes bewegliche Teil ein erstes Ende zum Berühren eines Objekts und ein zweites Ende aufweist, das fluoreszierendes oder phosphoreszierendes Material umfasst;
   ein Gehäuse zum Halten der beweglichen Teile, sodass die beweglichen Teile konfiguriert sind, sich innerhalb des Gehäuses parallel zueinander zu bewegen, um ihre jeweiligen Positionen gemäß einem Profil des berührten Objekts zu variieren;
   eine Lichtquelle (9) zum Anregen einer Lichtemission aus dem Material;
   eine Kamera (8), die konfiguriert ist, ein Bild der zweiten Enden der beweglichen Teile basierend auf der Lichtemission zu erhalten; und **dadurch gekennzeichnet, dass** die Vorrichtung weiter umfasst
   ein telezentrisches System zwischen den beweglichen Teilen und der Kamera (8).

2. Bildgebungsvorrichtung nach Anspruch 1, umfassend ein Fenster zwischen dem telezentrischen System und den beweglichen Teilen (1), wobei das Fenster für Wellenlängen des von der optischen Quelle und dem Material emittierten Lichts transparent ist.

3. Bildgebungsvorrichtung nach einem vorstehenden Anspruch, aufweisend einen Mechanismus zum Zurücksetzen der Positionen der beweglichen Teile, der Mechanismus umfassend eine Feder (13) und einen an dem Fenster angebrachten Schalter, wobei der Schalter konfiguriert ist, um, wenn aktiviert, zu bewirken, dass die Feder die Positionen zurücksetzt.

4. Bildgebungsvorrichtung nach einem vorstehenden Anspruch, wobei:

   die beweglichen Teile (1) Säulen, wie Stifte, umfassen, wobei die Teile vorzugsweise aus Metall, Kunststoff oder fluoreszierendem/phosphoreszierendem Material gebildet sind; und/oder
   das erste Ende des beweglichen Teils, das das Objekt berührt, ein drehbares Element, vorzugsweise ein Kugellager, umfasst.

5. Bildgebungsvorrichtung nach einem vorstehenden Anspruch, wobei:
   das Gehäuse umfasst:

   ein Array von Löchern, die jeweils den beweglichen Teilen (1) entsprechen, sodass sich die beweglichen Teile entlang einer Achse des Gehäuses frei in Längsrichtung bewegen können; und/oder
   einen Anschlag zum Limitieren der Bewegung der beweglichen Teile aus dem Gehäuse heraus, und/oder wobei die Bildgebungsvorrichtung eine Kappe umfasst, die konfiguriert ist, eine Barriere zwischen den beweglichen Teilen und dem Objekt bereitzustellen, wobei die Kappe vorzugsweise ein Einwegartikel ist.

6. Bildgebungsvorrichtung nach einem vorstehenden Anspruch, wobei das fluoreszierende oder phosphoreszierende Material eine homogene Schicht oder Beschichtung umfasst, vorzugsweise aufgesprüht oder gestrichen.

7. Bildgebungsvorrichtung nach einem vorstehenden Anspruch, wobei das erste Ende des beweglichen Teils (1), das das Objekt berührt, einen Sensor umfasst, der betriebsfähig ist, um Eigenschaften des zu messenden Objekts zu bestimmen, vorzugsweise den angelegten Druck oder die Temperatur.

8. Bildgebungsvorrichtung nach einem vorstehenden Anspruch, umfassend mindestens eine Feder (13), die an einem der beweglichen Teile und dem Gehäuse angebracht ist, wobei die Feder konfiguriert ist, Bestimmen der Kraft zu ermöglichen, die erzeugt wird, um das bewegliche Teil in seine jeweilige Position zu drücken.

9. Bildgebungsvorrichtung nach einem vorstehenden Anspruch, wobei die Kamera (8) eine Monochromkamera (8) ist.

10. Bildgebungssystem, umfassend die Bildgebungsvorrichtung nach einem vorstehenden Anspruch, wobei das System

    konfiguriert ist, für jedes bewegliche Teil, Teil (1), basierend auf dem Bild ein Unschärfemaß zu bestimmen und basierend auf der Bestimmung eine Entfernung oder Höhe des beweglichen Teils zu bestimmen.

11. Verwendung einer Bildgebungsvorrichtung nach mindestens Anspruch 7 der vorstehenden Ansprüche, wobei der Sensor ein Temperatursensor ist, der betriebsfähig ist, um die Oberflächentemperatur, vorzugsweise eines Menschen, zu messen, um entzündete Gelenkbereiche zu identifizieren.

12. Verwendung einer Bildgebungsvorrichtung nach Anspruch 8 oder einem davon abhängigen Anspruch der vorstehenden Ansprüche an einem Gewebe, um die Zugeigenschaften des Gewebes, das untersucht wird, zu beurteilen, wobei das Gewebe vom Objekt ist, wobei die Eigenschaften eine Gewebeeigenschaft umfassen, die eine von dem Gewebe erzeugte Kraft bestimmt, um das bewegliche Teil in seine jeweilige Position zu drücken, vorzugsweise wobei die Bildgebungsvorrichtung nach Anspruch 8 ist und der erzeugten Kraft ermöglicht wird, unter Verwendung der Feder gemessen zu werden.

13. Verfahren zum Modellieren eines Profils eines Objekts, wobei das Verfahren die Bildgebungsvorrichtung nach einem der Ansprüche 1 bis 9 verwendet, wobei das Bild auf einem Bild von beweglichen Teilen basiert ist, die eingeschränkt sind, um sich parallel zueinander zu bewegen, wobei das Bild von den beweglichen Teilen mit dem Objekt in Berührung steht, das Verfahren umfassend:

(a) Verwenden der Kamera (8), um ein Bild der beleuchteten Enden der beweglichen Teile zu erhalten;
(b) Bestimmen eines Unschärfemaßes für jedes bewegliche Teil;
(c) Bestimmen einer Höhe oder Entfernung des beweglichen Teils für jedes bewegliche Teil basierend auf dem bestimmten Unschärfemaß.

14. Verfahren nach Anspruch 13, umfassend Durchführen von:

einer Kalibrierungsprozedur unter Verwendung eines Objekts, das ein vorbestimmtes Profil aufweist, wobei die Kalibrierungsprozedur mindestens die Schritte (a) und (b) umfasst und basierend auf jedem bestimmten Unschärfemaß und dem vorbestimmten Profil eine Beziehung zwischen dem Unschärfemaß und der Entfernung oder Höhe mindestens eines beweglichen Teils erzeugt; und
Durchführen des Verfahrens unter Verwendung eines Objekts, das ein unbekanntes Profil aufweist, wobei der Schritt (c) basierend auf der erzeugten Beziehung durchgeführt wird.

15. Verfahren nach Anspruch 13, weiter umfassend Erhalten eines 3D-Bildes oder Modells von einem des Folgenden:

einem subkutanen Tumor;
einem von einem lebenden Organismus entnommenen Gewebe/Tumor; oder
einem subkutanen Geschwulst, vorzugsweise von einem Menschen, wobei das Geschwulst zum Beispiel eine Zyste ist und/oder an einem menschlichen Hals vorliegt, oder
um Objekte zu identifizieren und zu verarbeiten, die 3D-QR-Code oder Braille-Zeichen umfassen.

## Revendications

1. Dispositif d'imagerie pour mesurer un profil d'un objet, le dispositif comprenant :
une pluralité de parties mobiles (1),

dans lequel chaque partie mobile présente une première extrémité pour entrer en contact avec un objet et une seconde extrémité comprenant un matériau fluorescent ou phosphorescent ;
un boîtier pour tenir les parties mobiles de sorte que les parties mobiles sont configurées pour se déplacer parallèlement les unes aux autres au sein du boîtier afin de faire varier leurs positions respectives selon un profil de l'objet en contact ;
une source de lumière (9) pour stimuler l'émission de lumière du matériau ;
une caméra (8) configurée pour obtenir une image des secondes extrémités des parties mobiles sur la base de l'émission de lumière ; et **caractérisé en ce que** le dispositif comprend en outre
un système télécentrique entre les parties mobiles et la caméra (8).

2. Dispositif d'imagerie selon la revendication 1, comprenant une fenêtre entre le système télécentrique et les parties mobiles (1), dans lequel la fenêtre est transparente à des longueurs d'onde de la lumière émise par la source optique et le matériau.

**3.** Dispositif d'imagerie selon une quelconque revendication précédente, présentant un mécanisme pour réinitialiser les positions des parties mobiles, le mécanisme comprenant un ressort (13) et un interrupteur attaché à la fenêtre, l'interrupteur étant configuré pour, lorsqu'il est activé, amener le ressort à réinitialiser les positions.

**4.** Dispositif d'imagerie selon une quelconque revendication précédente, dans lequel :

les parties mobiles (1) comprennent des colonnes telles que des broches, les parties étant de préférence formées de métal, de plastique ou d'un matériau fluorescent/phosphorescent ; et/ou
la première extrémité de la partie mobile en contact avec l'objet comprend un élément rotatif, de préférence un roulement à billes.

**5.** Dispositif d'imagerie selon une quelconque revendication précédente, dans lequel :
le boîtier comprend :

un réseau de trous correspondant auxdites parties mobiles respectives (1) de sorte que les parties mobiles peuvent se déplacer librement longitudinalement le long d'un axe dudit boîtier ; et/ou
une butée pour restreindre le mouvement des parties mobiles hors du boîtier, et/ou dans lequel le dispositif d'imagerie comprend un capuchon configuré pour fournir une barrière entre les parties mobiles et l'objet, de préférence dans lequel le capuchon est jetable.

**6.** Dispositif d'imagerie selon une quelconque revendication précédente, dans lequel le matériau fluorescent ou phosphorescent comprend une couche ou un revêtement homogène, de préférence pulvérisé ou peint.

**7.** Dispositif d'imagerie selon une quelconque revendication précédente, dans lequel la première extrémité de la partie mobile (1) en contact avec l'objet comprend un capteur pouvant fonctionner pour déterminer des propriétés de l'objet à mesurer, de préférence la pression ou température appliquée.

**8.** Dispositif d'imagerie selon une quelconque revendication précédente, comprenant au moins un ressort (13) attaché à une dite partie mobile respective et à l'enceinte, le ressort étant configuré pour pouvoir déterminer la force générée pour pousser la partie mobile dans sa position respective.

**9.** Dispositif d'imagerie selon une quelconque revendication précédente, dans lequel la caméra (8) est une caméra monochrome (8).

**10.** Système d'imagerie comprenant le dispositif d'imagerie selon une quelconque revendication précédente, le système

étant configuré pour, pour chaque partie mobile, partie (1), déterminer sur la base de l'image une mesure de flou et déterminer une distance ou hauteur de la partie mobile sur la base de la détermination.

**11.** Utilisation d'un dispositif d'imagerie selon au moins la revendication 7 des revendications précédentes, dans lequel le capteur est un capteur de température pouvant fonctionner pour mesurer la température de surface, de préférence d'un humain, pour identifier des zones inflammées d'articulations.

**12.** Utilisation d'un dispositif d'imagerie selon la revendication 8 ou l'une quelconque des revendications qui en dépendent sur un tissu pour évaluer des propriétés de traction du tissu examiné, dans lequel le tissu représente un dit objet, les propriétés comprenant une propriété de tissu qui détermine une force générée par le tissu pour pousser une dite partie mobile à sa position respective, de préférence dans lequel le dispositif d'imagerie est selon la revendication 8 et la force générée peut être mesurée en utilisant un dit ressort.

**13.** Procédé de modélisation du profil d'un objet, le procédé utilisant le dispositif d'imagerie selon l'une quelconque des revendications 1 à 9, dans lequel l'image est basée sur une image de parties mobiles contraintes de se déplacer parallèlement entre elles, dans lequel l'image représente les parties mobiles en contact avec l'objet, le procédé comprenant :

(a) l'utilisation de la caméra (8) pour obtenir une image d'extrémités éclairées des parties mobiles ;
(b) la détermination, pour chacune desdites parties mobiles, d'une mesure de flou ;
(c) la détermination, pour chaque partie mobile, d'une hauteur ou distance de la partie mobile sur la base de la mesure de flou déterminée.

**14.** Procédé selon la revendication 13, comprenant la réalisation :

d'une procédure d'étalonnage en utilisant un dit objet présentant un profil prédéterminé, dans lequel la procédure d'étalonnage comprend au moins les étapes (a) et (b) et génère une relation entre la mesure du flou et la distance ou hauteur d'au moins une partie mobile, sur la base de chaque mesure de flou déterminée et du profil prédéterminé ; et
la réalisation du procédé en utilisant un objet présentant un profil inconnu, dans lequel l'étape (c) est réalisée sur la base de la relation générée.

**15.** Procédé selon la revendication 13, comprenant en outre l'obtention d'une image ou d'un modèle 3D de l'un quelconque parmi :

une tumeur sous-cutanée ;
des tissus/tumeurs retirés d'un organisme vivant ; ou
une bosse sous-cutanée, de préférence chez un humain, par exemple dans lequel la bosse est un kyste et/ou est sur un cou humain, ou
pour identifier et traiter des objets comprenant un code QR 3D ou des caractères Braille.

A

200x200          20x20          10x10          5x5

B

Multiple sampling
simulation

● = single
measurement

FIG. 1

FIG. 2

FIG. 3

C

**Step 1: Calibration sequence**

Repeat with objects at incremental heights

Device placed over a flat object, displacing the pins to a known height

Image(s) captured

Identify and create individual pin mask

Measure feature (e.g. blurriness)

**Step 2: Acquisition**

Device placed over object to be measured

Image(s) captured

Identify and create individual pin mask

Measure feature (e.g. blurriness)

Estimate pin height using calibration

Match inferred heights to xy coordinates to reconstruct the 3D profile

FIG. 3
(continued)

FIG. 4

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9423605 A1, Crew
- US 6160264 A, Rebiere
- US 2009112130 A1, Bengtson
- WO 2018109453 A1, Larkins

**Non-patent literature cited in the description**

- **PRINCE** ; **KENNEY** ; **HOWARD.** A pin-array method for capturing tissue deformation under defined pressure distributions and its application to prosthetic socket design. *Medical Engineering and Physics*, 2020, vol. 84, 136-143